# EUROPÄISCHE PATENTSCHRIFT

(11) **EP 0 700 491 B1**
(45) Veröffentlichungstag und Bekanntmachung des Hinweises auf die Patenterteilung: **10.09.1997**
(21) Anmeldenummer: 95914332.2
(22) Anmeldetag: 30.03.1995
(51) Int. Cl.: F16F 9/02, A61F 2/68

(54) **SCHWUNGPHASENSTEUERUNG FÜR EIN KÜNSTLICHES KNIEGELENK**
INERTIA PHASE CONTROL FOR ARTIFICIAL KNEE JOINTS
COMMANDE A PHASE D'INERTIE POUR ARTICULATIONS ARTIFICIELLES DU GENOU

(30) Priorität: 31.03.1994 DE 9405545 U
(43) Veröffentlichungstag der Anmeldung: 13.03.1996
(73) Patentinhaber: BIEDERMANN MOTECH GmbH, D-78054 Villingen-Schwenningen (DE)
(72) Erfinder: FITZLAFF, Gerhard, D-78054 VS-Schwenningen (DE)
(74) Vertreter: Prüfer, Lutz H., Dipl.-Phys.
(86) Internationale Anmeldenummer: EP9501192
(87) Internationale Veröffentlichungsnummer: WO9527156

(56) Entgegenhaltungen:
- WO-A-94/07442
- FR-A- 1 095 378
- GB-A- 982 527
- GB-A- 2 264 348
- US-A- 3 214 155

## Beschreibung

Die Erfindung betrifft eine Schwungphasensteuerung für ein künstliches Kniegelenk mit einem an seinem einen Ende mit einem Kopfteil und seinem anderen Ende mit einem Fußteil verschlossenen Zylinder sowie einem in diesem in axialer Richtung hin und her bewegbaren Kolben mit einer durch eine Bohrung im Kopfteil nach außen geführten Kolbenstange zum Verbinden mit einem Oberschenkelteil einer Prothese und einem durch den Kolben begrenzten kopfteilseitigen ersten Raum und einem fußteilseitigen zweiten Raum.

Eine derartige Vorrichtung ist aus der US-PS 5,062,857 bekannt.

Aufgabe der Erfindung ist es, eine Schwungphasensteuerung der eingangs beschriebenen Art zu schaffen, die für unterschiedlich schnelle Bewegungen eine gute Dämpfung ergibt und trotzdem einen einfachen Aufbau aufweist.

Diese Aufgabe wird durch eine Schwungphasensteuerung der eingangs beschriebenen Art gelöst, die dadurch gekennzeichnet ist, daß das Kopfteil einen Kanal aufweist, der die Umgebung mit dem ersten Raum verbindet und ein die Verbindung zwischen erstem Raum und Umgebung sperrendes Rückschlagventil aufweist, und daß der erste Raum und der zweite Raum durch einen Drosselweg miteinander verbunden sind und daß das Fußteil eine den zweiten Raum mit der Umgebung verbindende Belüftungsbohrung aufweist und eine den Kolben zum Kopfteil hin vorspannende erste Feder aufweist.

Mit dieser Lösung wird eine besonders einfache und leichte Schwungphasensteuerung geschaffen.

Gemäß einer besonderen Ausführungsform ist eine einen kürzeren Weg aufweisende zweite Feder für eine zweite Stufe der Vorspannung vorgesehen. Dadurch wird die Extension beschleunigt.

Weiterbildungen der Erfindung sind in den Unteransprüchen gekennzeichnet.

Weitere Merkmale und Zweckmäßigkeiten der Erfindung ergeben sich aus der Beschreibung von Ausführungsbeispielen anhand der Figuren.

Von den Figuren zeigen:
- Fig. 1: einen Querschnitt durch eine erste Ausführungsform;
- Fig. 2: einen Querschnitt durch eine zweite Ausführungsform in einer ersten Stellung; und
- Fig. 3: die zweite Ausführungsform in einer zweiten Stellung.

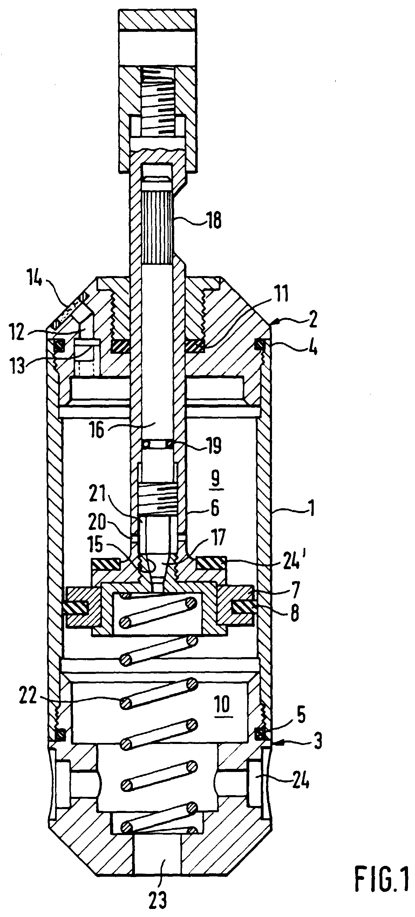

Wie am besten aus Fig. 1 ersichtlich ist, ist in dem zweiten Raum 10 eine koaxial ausgerichtete Druckfeder 22 vorgesehen, die mit ihrem einen Ende an einer Widerlagerfläche des Fußteiles 3 und mit ihrem gegenüberliegenden Ende an einer Widerlagerfläche des Kolbens 7 anliegt und die den Kolben in Richtung zum Kopfteil 2 hin vorspannt. Der zweite Raum 10 ist über eine Bohrung 23 mit der Umgebung verbunden. Ferner sind am Fußteil Bohrungen 24 zum Verbinden mit dem Unterschenkel vorgesehen. Der Kolben 7 weist auf seiner Oberseite ein ringförmiges Gummipolster 24' auf.

Im Betrieb wird die Flexionsbewegung durch die Feder 22 gedämpft. Die Extensionsbewegung hingegen wird einerseits durch die Feder 22 beschleunigt. Andererseits erfolgt eine Dämpfung durch das im ersten Raum 9 entstehende Luftkissen. Das Ausmaß der Dämpfung wird durch die axiale Stellung der Ventilstange 16 bzw. den Querschnitt des dadurch eingestellten Drosselweges bestimmt.

Wie am besten aus Fig. 1 ersichtlich ist, ist im Kopteil 2 ein Kanal in Form einer Bohrung 12 vorgesehen, die die Umgebung mit dem Inneren des ersten Raumes 9 verbindet. In der Bohrung ist ein als Flatterventil ausgebildetes Rückschlagventil 13 vorgesehen, welches so ausgebildet ist, daß es die Verbindung von der Umgebung zum ersten Raum freigibt, den Kanal aber in der entgegengesetzten Richtung sperrt. Die Außenseite der Bohrung ist durch einen Staubfilter 14 abgedeckt. Wie Fig. 1 erkennen läßt, weist der Kolben 7 eine koaxial ausgerichtete Bohrung 15 mit einem sich zum zweiten Raum 10 hin verjüngenden konischen Querschnitt auf, die in den zweiten Raum 10 mündet. Die Kolbenstange 6 ist hohl ausgebildet. In ihr ist eine sich koaxial erstreckende Ventilstange 16 angeordnet. Die Ventilstange 16 weist einen sich in axialer Richtung erstreckenden Schaft auf, dessen der Bohrung 15 zugewandtes Ende einen konischen Abschnitt 17 aufweist, dessen Kegelwinkel im wesentlichen gleich dem Winkel der konischen Bohrung 15 ist. Die Ventilstange ist in einem Gewinde geführt. An dem der Bohrung 15 abgewandten Ende ist die Ventilstange in einem gerändelten Bereich 18 zum Einstellen der Verschiebung in axialer Richtung zugänglich. Zwischen dem zugänglichen Bereich und dem konischen Abschnitt 17 ist eine Dichtung 19 vorgesehen, die eine Abdichtung nach außen bewirkt. Die Ventilstange weist in dem an den konischen Abschnitt 17 angrenzenden Bereich einen Durchmesser auf, der kleiner ist als der Innendurchmesser der Kolbenstange 6. Es ist eine einen nur geringen Luftwiderstand aufweisende Bohrung 20 in diesem Abschnitt der Kolbenstange 6 vorgesehen, die den ersten Raum 9 mit dem daran angrenzenden inneren 21 der Kolbenstange der Kolbenstange 6 verbindet. Durch Einstellen der Ventilstange 16 in axialer Richtung wird zwischen der Wandung der Bohrung 15 und dem konischen Abschnitt 17 ein Drosselkanal mit vorbestimmtem Querschnitt eingestellt, über den der erste Raum 9 mit dem zweiten Raum 10 verbunden ist.

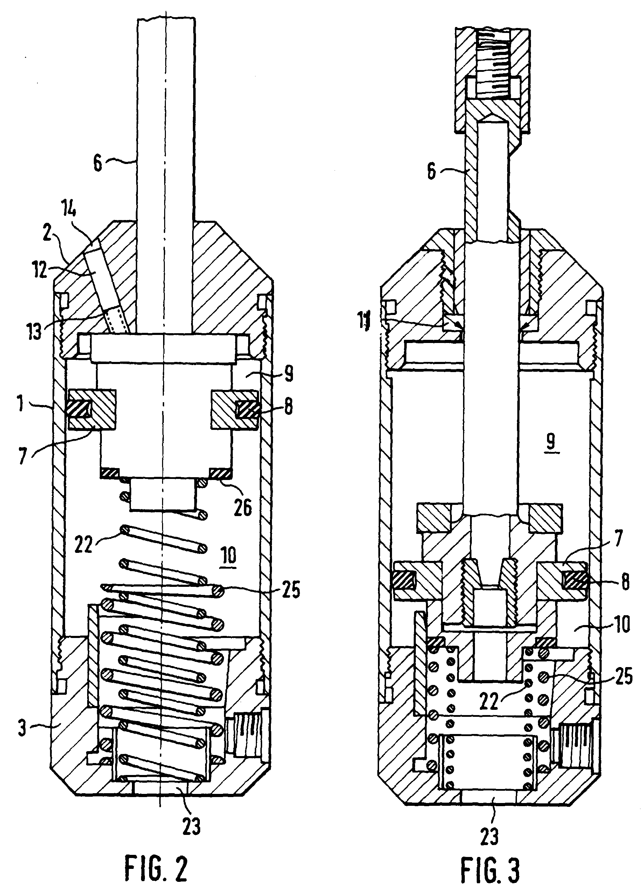

Die Figuren 2 und 3 zeigen schematisch angedeutet eine Schwungphasensteuerung, die mit der oben beschriebenen Ausführungsform übereinstimmt. Gleiche Teile sind mit den gleichen Bezugszeichen versehen. Der Kolben, die Kolbenstange und die Ventilstange sowie die Einstellmöglichkeiten sind nur schematisch angedeutet, haben aber den gleichen Aufbau wie bei der in Fig. 1 gezeigten Ausführungsform. Die in den Figuren 2 und 3 gezeigte Ausführungsform unterscheidet sich gegenüber der ersten Ausführungsform nur dadurch, daß eine zweite Druckfeder 25 vorgesehen ist. Diese liegt mit ihrem einen Ende an einem Widerlager des Fußteiles 3 an. Die zweite Druckfeder 25 weist in der aus Fig. 2 ersichtlichen Weise eine Länge auf, die nur über etwa der Hälfte des Abstandes zwischen einer Widerlagerfläche 26 des Kolbens 7 in der in Fig. 2 gezeigten obersten Extensionsstellung und der entgegengesetzten, in Fig. 3 gezeigten, untersten Flexionsstellung liegt.

Der Betrieb verläuft in der gleichen Weise wie bei der ersten Ausführungsform. Lediglich beginnt nach Ineingriffkommen der Widerlagerfläche 26 mit der zweiten Druckfeder 25 eine zusätzliche Flexionsdämpfung durch die hinzugeschaltete zweite Feder 25, die am Extensionsanfang diese beschleunigt.

Im Zylinderinneren ist ein von einer Kolbenstange 6 getragener Kolben 7 vorgesehen. Der Kolben 7 trennt mittels geeigneter Dichtungen 8 den Innenraum in dem Zylinder in einen kopfteilseitigen ersten Raum 9 und einen fußteilseitigen zweiten Raum 10. Die Kolbenstange 6 ist über eine koaxiale Bohrung im Kopfteil 2 nach außen geführt und weist an ihrem freien Ende eine Öse zum Verbinden mit einem Oberschenkelteil einer Prothese auf. Die Kolbenstange 6 ist in der koaxialen Bohrung gleitend gelagert. Durch Dichtungen 11 erfolgt eine Abdichtung des ersten Raumes 9 nach außen.

## Patentansprüche

1. Schwungphasensteuerung für ein künstliches Kniegelenk mit einem an seinem einen Ende mit einem Kopfteil (2) und an seinem anderen Ende mit einem Fußteil (3) verschlossenen Zylinder (1) sowie einem in diesem in axialer Richtung hin und her bewegbaren Kolben (7) mit einer durch eine Bohrung im Kopfteil (2) nach außen geführten Kolbenstange (6) zum Verbinden mit einem Oberschenkelteil einer Prothese und einem durch den Kolben (7) begrenzten kopfteilseitigen ersten Raum (9) und einen fußteilseitigen zweiten Raum (10), dadurch gekennzeichnet, daß das Kopfteil (2) einen Kanal (12) aufweist, der die Umgebung mit dem ersten Raum (9) verbindet und ein die Verbindung zwischen erstem Raum (9) und Umgebung sperrender Rückschlagventil (13) aufweist, daß der erste Raum (9) und der zweite Raum (10) durch einen Drosselweg miteinander verbunden sind, daß das Fußteil eine den zweiten Raum (10) mit der Umgebung verbindende Belüftungsbohrung (23) aufweist und eine den Kolben (7) zum Kopteil (2) hin vorspannende erste Feder (22) aufweist.

2. Schwungphasensteuerung für ein künstliches Kniegelenk nach Anspruch 1, dadurch gekennzeichnet, daß der Querschnitt des Drosselweges einstellbar ausgebildet ist.

3. Schwungphasensteuerung für ein künstliches Kniegelenk nach Anspruch 1 oder 2, dadurch gekennzeichnet, daß der Drosselweg als eine koaxial ausgerichtete Bohrung (15) mit einem sich zum zweiten Raum (10) hin verjüngenden konischen Querschnitt und einer damit zusammenwirkenden, im Inneren der Kolbenstange (6) geführten und in axialer Richtung verschiebbaren Ventilstange (16) gebildet ist.

4. Schwungphasensteuerung für ein künstliches Kniegelenk nach einem der Ansprüche 1 bis 3, dadurch gekennzeichnet, daß eine einen kürzeren Weg aufweisende zweite Feder (25) für eine zweite Stufe der Vorspannung vorgesehen ist.

## Claims

1. An inertia phase control system for an artificial knee joint with a cylinder (1) closed at one of its ends by a head-piece (2) and at its other end by a base-piece (3) as well as a piston (7) which is movable to and fro inside the said cylinder in an axial direction and has a piston rod (6) guided towards the outside through a bore in the head-piece (2) and intended for connection to the upper thigh part of an artifical limb and which has a first chamber (9) situated at the head-piece end and delimited by the piston (7) and a second chamber (10) situated at the base-piece end, characterized in that the head-piece (2) has a duct (12) which connects the surrounding environment to the first chamber (9) and has a non-return valve (13) closing off the connection between first chamber (9) and surrounding environment, in that the first chamber (9) and the second chamber (10) are connected to one another by a constriction passage, in that the base-piece has a ventilation bore (23) connecting the second chamber (10) with the surrounding environment and a first spring (22) pretensioning the piston (7) towards the head-piece (2).

2. Inertia phase control system for an artificial knee joint according to Claim 1, characterized in that the cross-section of the constriction passage is designed so as to be adjustable.

3. Inertia phase control system for an artificial knee joint according to Claim 1 or Claim 2, characterized in that the constriction passage is formed as a coaxially aliqned bore (15) with a conical cross-section tapering towards the second chamber (10) and a valve rod (16) cooperating therewith, guided inside the piston rod (6) and displaceable in the axial direction.

4. Inertia phase control system for an artificial knee joint according to one of Claims 1 to 3, characterized in that a second spring (25) having a shorter travel is provided for a second stage of the pretension action.

## Revendications

1. Commande à phase d'inertie destinée à une articulation artificielle du genou, comprenant un cylindre (1) fermé à une extrémité par une tête (2) et à l'autre extrémité par une base (3), ainsi qu'un piston (7) susceptible d'être déplacé dans le sens axial, lequel est muni d'une tige de piston (6), qui est guidée vers l'extérieur à travers une forure réalisée dans la tête (2) et est destinée à être reliée à un fémur d'une prothèse, et comprenant une première chambre (9) disposée près de la tête et délimitée par le piston (7) et une deuxième chambre (10) disposée près de la base, caractérisée en ce que la tête (2) est munie d'un conduit (12), par lequel la première chambre (9) communique avec l'environnement et dans lequel est montée une soupape de retenue (13) qui obture la liaison entre la première chambre (9) et l'environnement, en ce que la première chambre (9) et la deuxième chambre (10) sont reliées entre elles par une voie d'étranglement, en ce que la base est munie d'une forure d'aération (23) faisant communiquer la deuxième chambre (10) avec l'environnement et d'un premier ressort (22) destiné à précontraindre le piston (7) vers la tête (2).

2. Commande à phase d'inertie destinée à une articulation artificielle du genou selon la revendication 1, caractérisée en ce que la section de la voie d'étranglement est conçue de manière à pouvoir être régulée.

3. Commande à phase d'inertie destinée à une articulation artificielle du genou selon la revendication 1 ou 2, caractérisée en ce que la voie d'étranglement est formée par une forure (15), orientée dans le sens coaxial, comprenant une section conique, qui se rétrécit en allant vers la deuxième chambre (10), et une tige de soupape (16), susceptible d'être déplacée dans le sens axial et guidée à l'intérieur de la tige de piston (6), qui agit en même temps que ladite section conique.

4. Commande à phase d'inertie destinée à une articulation artificielle du genou selon l'une quelconque des revendications 1 à 3, caractérisée en ce qu'il est prévu de monter un deuxième ressort (25) présentant une plus petite course, pour une deuxième étape de précontrainte.
